# EUROPEAN PATENT APPLICATION

(11) **EP 4 501 225 A1**
(43) Date of publication of application: **05.02.2025**
(21) Application number: 23774002.2
(22) Date of filing: 24.03.2023
(51) Int. Cl.: A61B 5/08, A61B 5/113

(54) **LUNG FUNCTION DETECTION METHOD, SYSTEM AND APPARATUS, AND COMPUTER DEVICE AND STORAGE MEDIUM**

(30) Priority: 25.03.2022 CN 202210302918
(71) Applicant: Shenzhen Huayi Medical Technologies Co., Ltd., Shenzhen, Guangdong 518051 (CN)
(72) Inventor: SONG, Weidong, Shenzhen, Guangdong 518051 (CN); HUANG, Yi, Shenzhen, Guangdong 518051 (CN); HU, Wei, Shenzhen, Guangdong 518051 (CN); LEI, Junjie, Shenzhen, Guangdong 518051 (CN); GONG, Yan, Shenzhen, Guangdong 518051 (CN)
(74) Representative: Hoffmann Eitle
(86) International application number: PCT/CN2023/083636
(87) International publication number: WO 2023/179757

(57) **Abstract**

The present application relates to a lung function detection method, system and apparatus, and to a computer device and a storage medium. The method comprises: acquiring chest and abdomen surface point cloud data of a subject under test within a preset time period by means of a biological detection sensor; analyzing the chest and abdomen surface point cloud data, and determining lung ventilation change information of the subject under test; and according to the lung ventilation change information, determining a lung function detection result of the subject under test. The biological detection sensor is arranged within a preset range around the subject under test but does not come in contact with the subject under test.

## Description

### CROSS-REFERENCE TO RELATED APPLICATIONS

This application claims priority to Chinese Patent Application No. 202210302918.6, entitled "LUNG FUNCTION DETECTION METHOD, SYSTEM AND APPARATUS, AND COMPUTER DEVICE AND STORAGE MEDIUM" and filed with the China Patent Office on March 25, 2022, the entire contents of which are incorporated herein by reference.

### TECHNICAL FIELD

The present application relates to the field of medical detection technologies, and in particular, to a lung function detection method, system and apparatus, and a computer device and a storage medium.

### BACKGROUND

Lung function detection is an effective means of examining patients' lung diseases and is clinically used to examine and diagnose chronic obstructive pulmonary diseases, respiratory stenosis, and other diseases.

In the related art, a patient needs to blow air into a blowing nozzle of a detection device with his/her mouth, and a lung condition of the patient is examined through changes in an amount of air blown over time. In this process, there is a need to clamp the patient's nose with a clamp to prevent air leakage from the nose, so that the patient can breathe completely through the mouth and follow a breathing pattern of "normally breathe - inhale to a maximum extent - exhale to a maximum extent", and a doctor determines a lung function detection result of the patient by analyzing a lung ventilation change curve of the patient over time.

### SUMMARY

In a first aspect, the present application provides a lung function detection method. The method includes:
acquiring chest and abdomen surface point cloud data of a subject under test within a preset time period by means of a biological detection sensor; wherein the biological detection sensor is arranged within a preset range around the subject under test but does not come in contact with the subject under test;
analyzing the chest and abdomen surface point cloud data, and determining lung ventilation change information of the subject under test; and
according to the lung ventilation change information, determining a lung function detection result of the subject under test.

In a second aspect, the present application further provides a lung function detection system, wherein the system includes at least one biological detection sensor and a lung function detection device, the lung function detection device being connected to the at least one biological detection sensor;
the biological detection sensor being configured to collect chest and abdomen surface point cloud data of a subject under test within a preset time period; and
the lung function detection device being configured to analyze the chest and abdomen surface point cloud data collected by the biological detection sensor, determine lung ventilation change information of the subject under test, and according to the lung ventilation change information, determine a lung function detection result of the subject under test.

In a third aspect, the present application further provides a lung function detection apparatus. The apparatus includes:
an acquisition module configured to acquire chest and abdomen surface point cloud data of a subject under test within a preset time period by means of a biological detection sensor; wherein the biological detection sensor is arranged within a preset range around the subject under test but does not come in contact with the subject under test;
an analysis module configured to analyze the chest and abdomen surface point cloud data, and determine lung ventilation change information of the subject under test; and
a first determination module configured to, according to the lung ventilation change information, determine a lung function detection result of the subject under test.

In a fourth aspect, the present application further provides a lung function detection device. The lung function detection device includes a memory and a processor, the memory storing a computer program, wherein the processor, when executing the computer program, implements steps in any one of the method embodiments in the first aspect above.

In a fifth aspect, the present application further provides a computer-readable storage medium. The computer-readable storage medium has a computer program stored therein, wherein the computer program, when executed by a processor, implements steps in any one of the method embodiments in the first aspect above.

In a sixth aspect, the present application further provides a computer program product. The computer program product includes a computer program, wherein the computer program, when executed by a processor, implements steps in any one of the method embodiments in the first aspect above.

Details of one or more embodiments of the present application are set forth in the following accompanying drawings and descriptions. Other features, objectives, and advantages of the present application become obvious with reference to the specification, the accompanying drawings, and the claims.

### BRIEF DESCRIPTION OF THE DRAWINGS

In order to better describe and illustrate embodiments and/or examples of those inventions disclosed herein, reference may be made to one or more accompanying drawings. However, additional details or examples used to describe the accompanying drawings should not be considered as limitations on the scope of any of inventions and creations, currently described embodiments, and preferred manners of the present application.
FIG. 1 is a diagram of an application environment of a lung function detection method according to an embodiment;
FIG. 2 is a schematic flowchart of a lung function detection method according to an embodiment;
FIG. 3 is a schematic flowchart of acquiring chest and abdomen surface point cloud data according to an embodiment;
FIG. 4 is a schematic flowchart of acquiring lung ventilation change information according to an embodiment;
FIG. 5 is a schematic flowchart of a lung function detection method according to another embodiment;
FIG. 6 is a structural block diagram of a lung function detection system according to an embodiment;
FIG. 7 is a schematic flowchart of a sleep quality monitoring method according to an embodiment;
FIG. 8 is a structural block diagram of a lung function detection apparatus according to an embodiment; and
FIG. 9 is a diagram of an internal structure of a lung function detection device according to an embodiment.

### DETAILED DESCRIPTION

In order to make the objectives, technical solutions, and advantages of the present application clearer, the present application will be further described in detail below in conjunction with the accompanying drawings and embodiments. It should be understood that the specific embodiments described herein are intended only to explain the present application and are not intended to limit the present application.

Breathing ventilation refers to a flow of gas entering and exiting a lung during breathing of a human body, which may reflect a ventilation function of the lung and may also be used to detect some special diseases. However, existing breathing ventilation detection technologies for medical treatment require a subject under test to wear or come into contact with a special device, which may bring great inconvenience to the subject under test, and may waste test materials and be at a risk of contact infection, resulting in limitations to the use of the detection technologies.

Generally, the subject under test is detected in a contact manner during lung function detection. Mainstream methods include: detection by using chest and abdomen strap pressure, detection of a nasal airflow state by using a small sensor, and detection of lung ventilation by blowing air into a blowing nozzle. A main principle of the method of detection by using chest and abdomen strap pressure is: to tie a strap for detection on the chest and abdomen and monitor rises and falls of the chest and abdomen of the subject under test during the breathing, so as to detect a lung function of the subject under test in real time according to a breathing state thereof. A main principle of the method of detection of a nasal airflow state by using a small sensor is: to obtain a current breathing state of the subject under test by monitoring a nasal cavity airflow velocity and an airflow direction, so as to detect the lung function of the subject under test in real time according to a breathing state thereof. A main principle of the method of detection of lung ventilation by blowing air into a blowing nozzle is: to blow, by the subject under test, air into a blowing nozzle of a detection device and detect lung ventilation of the subject under test through changes in an amount of air blown over time, so as to analyze the lung function of the subject under test according to changes in the lung ventilation.

However, in the above lung function detection manners, the subject under test may experience discomfort if wearing these detection devices for a long time period. Further, during the detection, these devices may interfere with the breathing process of the subject under test and affect accuracy of measurement results. In addition, in the related art, there are relatively high requirements for the patents' breathing during the detection, and it is difficult for many patients to breathe accurately according to a breathing pattern, resulting in inaccurate detection results. Moreover, the method of detection by blowing air into a blowing nozzle is at a risk of cross infection.

Based on at least one detect in the contact-based lung function detection, the present application provides a lung function detection method, system and apparatus, and a computer device and a storage medium, in which a lung function of a subject under test is detected in a non-contact manner, and during the detection, there are relatively low testing requirements for the subject under test. The lung function detection method has better applicability. Compared with contact detection which is easy to introduce detection errors during the detection, the detection method in the present application can improve accuracy of detection results.

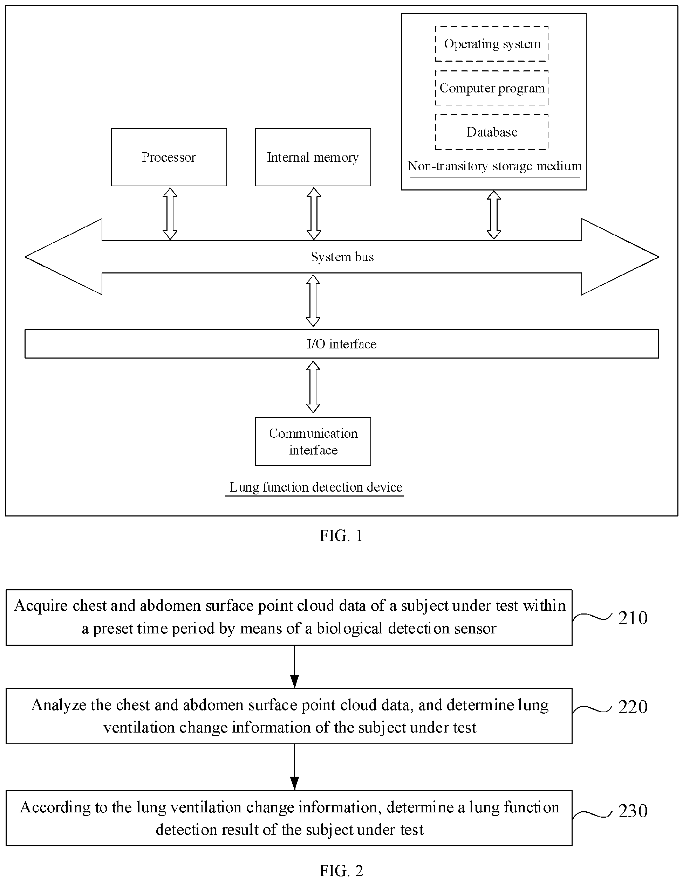

In a possible implementation, the lung function detection method provided in the present application may be applied to an application environment as shown in FIG. 1.

A lung function detection device in the application environment includes, but is not limited to, a personal computer, a notebook computer, a smartphone, a tablet computer, a portable wearable device, a medical detection device, and the like, which is not limited in the embodiments of the present application. A processor of the lung function detection device is configured to provide data analysis and processing capabilities. A memory of the lung function detection device includes a non-transitory storage medium and an internal memory. The non-transitory storage medium stores an operating system, a computer program, and a database. The internal memory provides an environment for running of the operating system and the computer program in the non-transitory storage medium. The database of the lung function detection device is used to store data such as detection indexes in lung function detection, diagnostic bases for lung-related diseases, and historical detection data of the subj ect under test. A network interface of the lung function detection device is used to communicate with other external devices through a network connection. The computer program is executed by the processor to implement a lung function detection method.

The technical solutions of the embodiments of the present application and how the technical solutions of the embodiments of the present application solve the above technical problems will be specifically described in detail below through embodiments and in conjunction with the accompanying drawings. The following specific embodiments may be mutually combined, and same or similar concepts or processes may not be repeatedly described in some embodiments. Obviously, the described embodiments are some of rather than all of the embodiments of the present application.

It is to be noted that a lung function detection method provided in the embodiments of the present application may be performed by the above lung function detection device, or may be further performed by a lung function detection apparatus. The apparatus may be implemented as part or all of the lung function testing device through software, hardware, or a combination of software and hardware.

In an embodiment, as shown in FIG. 2, a lung function detection method is provided. Description is based on an example in which the method is applied to the lung function detection device in FIG. 1, including the following steps.

In step 210, chest and abdomen surface point cloud data of a subject under test within a preset time period is acquired by means of a biological detection sensor.

The biological detection sensor is arranged within a preset range around the subject under test but does not come in contact with the subject under test. The preset range may be determined based on a sensing range of the biological detection sensor. For example, if the preset range is 2 m, the biological detection sensor is arranged around the subject under test and is only 2 m away from the subject under test.

It is to be noted that the subject under test may be a human or another animal body. The lung function detection process may be performed continuously or within a corresponding time period. The preset time period may be any preset time period based on an actual situation of the subject under test.

In order to detect a lung function of the subject under test without contact, the biological detection sensor may be a sensor that may visually collect information about rises and falls of a body surface of the subject under test with breathing, such as a radar (e.g., a millimeter-wave radar), a depth camera (e.g., an RGBD camera), or an infrared thermal imager, which is not specifically limited in the present application.

As an example, the biological detection sensor in the present application may be a radar. When performing lung function detection, the radar is arranged in front of the chest and abdomen of the subject under test.

Preferably, the radar serving as the biological detection sensor may be a millimeter-wave radar. An electromagnetic wave with a wavelength of 1 to 10 mm is called a millimeter wave, which is located in a wavelength range where microwaves and far-infrared waves overlap and therefore has characteristics of two spectrums. Compared with a light wave, the millimeter wave has less attenuation when propagating through an atmospheric window and is less affected by natural light and thermal radiation sources. The millimeter wave has the following advantages. (1) The millimeter wave has an extremely wide bandwidth. It is generally considered that the millimeter wave is within a frequency range from 26.5 GHz to 300 GHz and has a bandwidth up to 273.5 GHz, which is more than 10 times the entire bandwidth from DC to microwave. Even in consideration of atmospheric absorption, only four main windows can be used in the case of propagation in the atmosphere, but a total bandwidth of the four windows may reach 135 GHz, which is five times a sum of bandwidths of all bands below microwave. This is undoubtedly very attractive today when frequency resources are in shortage. (2) The millimeter wave has a narrow beam. Under a same antenna size, a beam of the millimeter wave is much narrower than that of the microwave. For example, a 12-cm antenna has a beam width of 18 degrees at 9.4 GHz, but a wave velocity width is only 1.8 degrees at 94 GHz. Therefore, small targets that are closer can be distinguished or details of a target can be observed more clearly. (3) Compared with a laser, propagation of the millimeter wave is much less affected by climate and may be considered to have all-weather characteristics. (4) Compared with the microwave, a millimeter wave component is much smaller in size, so a millimeter wave system is easier to miniaturize. (5) The millimeter wave can penetrate ordinary clothing and directly measure a body surface without requiring the subject to remove clothing.

For example, the biological detection sensor is a millimeter-wave radar. In a possible implementation, an implementation process of step 210 may be as follows. In response to a triggered detection request, the millimeter-wave radar preset around the subject under test uses a millimeter wave to illuminate the subject under test, and the subject under test may reflect a corresponding millimeter wave. The chest and abdomen of the subject under test may have corresponding rises and falls during breathing. Data of the rises and falls of the chest and abdomen can be accurately captured based on the reflected millimeter wave (such as phase information). The lung function detection device performs subsequent step 220 after receiving chest and abdomen surface point cloud data collected by the millimeter-wave radar.

The chest and abdomen surface point cloud data may accurately describe rises and falls of the chest and abdomen of the subject under test within the preset time period caused by breathing.

Optionally, during lung function detection on the subject under test, breathing may cause small movement of a trunk, and movement of the back of the subject under test may describe the movement of the trunk. Based on this, the radar serving as the biological detection sensor may include a primary radar and a secondary radar, the primary radar is arranged in front of the chest and abdomen of the subject under test, and the secondary radar is arranged behind the back of the subject under test.

It is to be noted that, when both the primary radar and the secondary radar are deployed, the lung function detection device is required to eliminate data interference between the primary radar and the secondary radar when receiving surface point cloud data collected by the two radars, to ensure accuracy of data collected by each radar.

As an example, when the data interference between the primary radar and the secondary radar is eliminated, time sharing of a transmitted waveform may be used (i.e., the primary radar and the secondary radar emit radar waves based on different time points, and emission times thereof are staggered), or different encoding forms (e.g., binary phase modulation) may be used for a primary radar waveform and a secondary radar waveform so that after an antenna of the lung function detection device receives signals of the two radars, the signals can be distinguished based on encoding schemes. Certainly, the interference may alternatively be eliminated in other manners, which is not limited in this embodiment.

In actual application, if the lung function detection device and the method provided in the present application are applied to a respiratory medicine department of a hospital, the primary radar and the secondary radar are preset in a corresponding detection space. Distances between the primary and secondary radars and the subject under test may be the same or different. During detection, the subject under test is just required to sit quietly within detection ranges of the primary radar and the secondary radar. If the lung function detection device and the method provided in the present application are applied to home detection, the primary radar and the secondary radar may be arranged at corresponding positions in a room, and during the detection, the subject under test sits quietly within the detection ranges of the primary radar and the secondary radar. In this case, chest and abdomen surface point cloud data collected by the primary radar may be corrected based on back surface point cloud data collected by the secondary radar to reduce an error introduced by the primary radar during breath detection, making the chest and abdomen surface point cloud data more accurate.

In addition, if there are a plurality of subjects under test, positions of the subjects under test may be scanned and searched, that is, positions of the subjects under test are obtained, a beam irradiation direction of the millimeter-wave radar is adjusted based on the positions of the subjects under test, and at the same time, chest and abdomen surface point cloud data of the plurality of subjects under test is detected, to achieve simultaneous detection of lung functions of the plurality of subjects under test.

In step 220, the chest and abdomen surface point cloud data is analyzed, and lung ventilation change information of the subject under test is determined.

The lung ventilation change information includes a plurality of lung function detection indexes, for example, a peak expiratory flowrate (PEF), a forced vital capacity (FVC), a slow vital capacity (SVC), a forced expiratory volume in one second (FEV1), and FEV1/FVC. The FVC is a total volume of air exhaled, and the FEV1 is a volume of air exhaled in the first second of exhalation, which is used to denote resistance of an airway to breathing.

In a possible implementation, an implementation process of step 220 may involve: analyzing the chest and abdomen surface point cloud data of the subject under test, and determining changes in a lung volume corresponding to the chest and abdomen surface point cloud data during the breathing, thereby determining the lung ventilation change information according to the changes in the lung volume of the subject under test.

In step 230, according to the lung ventilation change information, a lung function detection result of the subject under test is determined.

The lung function detection result may include detection values of the lung function detection indexes, standard value ranges corresponding to the lung function detection indexes, and a diagnostic result. The diagnostic result may include: there is no abnormality in the lung function of the subject under test; or according to the lung ventilation change information, the subject under test may have a certain lung disease.

In a possible implementation, an implementation process of step 230 may involve: acquiring detection values of the lung function detection indexes included in the lung ventilation change information and standard value ranges corresponding to the lung function detection indexes; and analyzing whether the detection values of the detection indexes are within the corresponding standard value ranges, and then outputting the lung function detection result of the subject under test based on medical knowledge.

As an example, the subject under test is a human body. Regarding the lung function detection indexes in the above example, generally, an average PEF of healthy men and women is about 10 L/s and 8 L/s respectively, while the PEF of asthma patients may be as low as 5 L/s; and generally, an average FEV1 of healthy people is about 3.75 L/s, while the FEV1 of chronic obstructive pulmonary disease may be as low as 2 L/s. Typically, a decrease in the FVC indicates a worsening condition. An average FVC of healthy men and women is about 5.25 L/s and 3.75 L/s, while the FVC for chronic obstructive pulmonary disease may be as low as 2.5 L/s. FEV1/FVC is used as a reference ratio. Generally, a ratio for healthy people should be greater than 80%, while for asthma patients, the ratio may be as low as 50% to 60%.

In order to further ensure accuracy of the lung function detection result, the standard value ranges of the lung function detection indexes may also be slightly adjusted with reference to different health standards according to age, gender, race, and the like of the subject under test, which is not limited in this embodiment.

Further, alarm information is sent if the lung function detection result of the subject under test is abnormal, and the alarm information is used to indicate that at least one detection index in the lung function detection result is abnormal.

As an example, the alarm information may be sent through a voice alarm, a change in a color of an indicator light, an APP, or the like. Moreover, according to degrees of danger, an alarm is sent to a family member of the subject under test and a hospital where the subject under test is located, so that the subject under test can be treated in a timely and effective manner in the case of a critical lung disease.

In addition, based on the above lung function detection method, a breathing condition of the subject under test may also be tracked, and abnormal alarms may be sent according to changes in the lung function detection result within a monitoring time period.

As an example, if there are obvious lung function change trends in the subject under test over a period of time, these lung function change trends may be signs of changes in certain health conditions and require attention to prevent or predict possible lung diseases in advance. Therefore, when the lung function detection result of the subject under test shows significant changes compared with historical data, even if all the lung function detection indexes are within the standard value ranges, abnormal alarm information is also required to feed back the change trend to the user or the doctor.

In addition, the above lung function detection method may also be used to detect a heart function. Generally, heartbeat and breathing frequencies of an organism are different. Taking a human body as an example, a heartbeat of the human body is about 60 to 80 beats per minute, and breathing is about 18 to 20 times per minute. Assuming that a heartbeat of a certain human body is calculated at 80 beats per minute, a frequency thereof is about 1.34; if breathing is calculated at 18 beats per minute, the frequency thereof is about 0.3. There is a big difference between the heartbeat frequency and the breathing frequency, and both may cause rises and falls in a chest and abdomen surface of the human body. Therefore, an implementation similar to that in the present application may be used to detect a cardio-pulmonary function of the subject under test and output a corresponding cardio-pulmonary function detection result.

In the embodiments of the present application, chest and abdomen surface point cloud data of a subject under test within a preset time period is acquired by means of a biological detection sensor; the chest and abdomen surface point cloud data is analyzed, and lung ventilation change information of the subject under test is determined; and according to the lung ventilation change information, a lung function detection result of the subject under test is determined. The biological detection sensor is arranged within a preset range around the subject under test but does not come in contact with the subject under test. In the present application, during the lung function detection on the subject under test, the lung ventilation change information is determined through the chest and abdomen surface point cloud data of the subject under test collected by the biological detection sensor. Compared with the related art in which the lung ventilation change information of the subject under test is detected by blowing air into a blowing nozzle, there are no requirements for the breathing pattern of the subject under test, which prevents detection errors caused by an unsatisfactory breathing rhythm of the subject under test, thereby improving accuracy of the detection result. In addition, the biological detection sensor in the present application is not in contact with the subject under test, and is not required to be in direct contact with the subject under test during the detection. For scenarios in hospitals where lung function detection is required to be performed sequentially on a plurality of subjects under test, non-contact detection may reduce a risk of cross infection and is safer.

Based on the above embodiments, during actual detection, for the subject under test, original data collected by the radar is body surface point cloud data of the subject under test. The body surface point cloud data is required to be screened to determine the chest and abdomen surface point cloud data that represents rises and falls of the chest and abdomen of the subject under test with breathing.

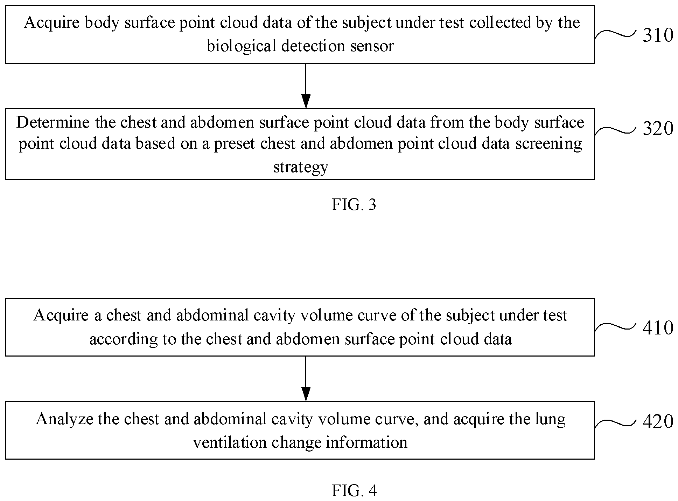

In an embodiment, as shown in FIG. 3, an implement process of acquiring chest and abdomen surface point cloud data of a subject under test within a preset time period by means of a biological detection sensor in step 210 may include the following steps.

In step 310, body surface point cloud data of the subject under test collected by the biological detection sensor is acquired.

It is to be noted that based on the setting position of the biological detection sensor and a detection range thereof, initially collected data is generally the body surface point cloud data of the subject under test, which is difficult to be accurate to the chest and abdomen of the subject under test.

The body surface point cloud data includes position information of respective points in a body surface of the subject under test, and body surface point cloud data collected within the preset time period may describe rises and falls of the body surface of the subject under test.

In step 320, the chest and abdomen surface point cloud data is determined from the body surface point cloud data based on a preset chest and abdomen point cloud data screening strategy.

The preset chest and abdomen point cloud data screening strategy includes distances between the point cloud points, moving velocities of the point cloud points, and three-dimensional spatial position coordinates of the point cloud points.

In a possible implementation, an implementation process of step 320 may involve: screening, based on the distances between the point cloud points and the moving velocities of the point cloud points, point cloud points at moving velocities of almost 0 from the body surface point cloud data, to eliminate point cloud points outside the chest and abdomen; and screening, from the body surface point cloud data, point cloud points whose three-dimensional spatial coordinates are much larger than an actual distance between the subject under test and the biological detection sensor, to eliminate point cloud points that represent a background of an environment where the subject under test is located. In this way, after irrelevant points are eliminated, the remaining point cloud points are the chest and abdomen surface point cloud data.

In another possible implementation, the implementation process of step 320 may involve: screening, based on the distances between the point cloud point, the moving velocities of the point cloud points, and the three-dimensional spatial position coordinates of the point cloud points, chest and abdomen point cloud points that represent changes in the chest and abdomen of the subject under test from the body surface point cloud data, to obtain the chest and abdomen surface point cloud data.

It is to be noted that step 310 and step 320 may be performed by the biological detection sensor. That is, a processing unit of the biological detection sensor analyzes the collected body surface point cloud data to determine the chest and abdomen surface point cloud data, and sends the determined chest and abdomen surface point cloud data to the lung function detection device.

Step 310 and step 320 may alternatively be performed by the lung function detection device. That is, the biological detection sensor sends the collected body surface point cloud data to the lung function detection device, and the lung function detection device analyzes the body surface point cloud data to determine the chest and abdomen surface point cloud data.

In this embodiment, based on the preset chest and abdomen point cloud data screening strategy, the chest and abdomen surface point cloud data is determined from the body surface point cloud data of the subject under test collected by the biological detection sensor, which improves accuracy of the chest and abdomen surface point cloud data, so that the chest and abdomen surface point cloud data obtained after elimination of irrelevant point cloud data can accurately and effectively describe rises and falls of the chest and abdomen of the subject under test with breathing.

Further, after the chest and abdomen surface point cloud data is acquired, the lung function detection device may analyze the chest and abdomen surface point cloud data, and determines the lung ventilation change information of the subject under test.

In an embodiment, as shown in FIG. 4, an implementation process of analyzing the chest and abdomen surface point cloud data, and determining lung ventilation change information of the subject under test in step 220 may include the following steps.

In step 410, a chest and abdominal cavity volume curve of the subject under test is acquired according to the chest and abdomen surface point cloud data.

The chest and abdominal cavity volume curve represents changes in a chest and abdominal cavity volume of the subject under test over time.

In a possible implementation, an implementation process of step 410 may involve: reconstructing a chest and abdomen surface based on the chest and abdomen surface point cloud data, and acquiring a chest and abdomen surface model; and acquiring the chest and abdominal cavity volume curve of the subject under test according to the chest and abdomen surface model.

When the chest and abdomen surface is reconstructed, based on the chest and abdomen surface point cloud data, three-dimensional coordinates of the point cloud points may be interpolated based on various surface functions to generate denser point clouds and surface meshes. Alternatively, the chest and abdomen surface may be reconstructed using a trained model. For example, body surface information may be obtained using a skinned multi-person linear model (SMPL).

As an example, each point cloud point in the chest and abdomen surface point cloud data includes coordinate information of a three-dimensional space thereof. Therefore, a surface expression of a region where the point cloud points are located may be fit by using three-dimensional coordinate information of adjacent point cloud points in a polynomial or B-spline manner, to obtain a surface expression of the chest and abdomen surface, that is, a model of the reconstructed chest and abdomen surface.

In another example, the SMPL is a three-dimensional human body model trained in advance through a large amount of data and deep learning, which may reconstruct body surface and posture information of a human body after three-dimensional coordinates of several points on a surface of the human body are inputted. In the present application, the chest and abdomen surface model may be obtained by substituting the three-dimensional coordinates of the point cloud points in the chest and abdomen surface point cloud data into the SMPL.

Further, based on the obtained chest and abdomen surface model, changes in the chest and abdomen surface over time are tracked, a change curve of the chest and abdominal cavity volume over time is calculated, and the chest and abdominal cavity volume curve of the subject under test is obtained.

In step 420, the chest and abdominal cavity volume curve is analyzed, and the lung ventilation change information is acquired.

The lung ventilation change information may alternatively be presented in the form of an image. That is, a curve of changes in lung ventilation of the subject under test over time is generated after the chest and abdominal cavity volume curve is analyzed. Moreover, time periods corresponding to the two curves are as long as the preset time period.

In a possible implementation, an implementation process of step 420 may involve: acquiring a conversion relationship between the chest and abdominal cavity volume and a lung volume; determining lung volume change information of the subject under test according to the chest and abdominal cavity volume curve and the conversion relationship; and determining the lung ventilation change information according to the lung volume change information.

It is to be noted that breathing includes chest breathing and abdominal breathing. That is, pulling down a diaphragm or expanding a thorax may cause the lung to be expanded, which is inhalation, and otherwise is exhalation. Therefore, different changes in the chest and abdomen surface may cause changes in a gas volume of the lung (referred to as a lung volume). Moreover, the corresponding relationship of this change may vary from person to person. Different subjects under test may have different changes in lung volumes during breathing.

Therefore, in order to analyze changes in the lung volume through changes in the chest and abdomen volume, there is a need to calibrate the lung function detection device in the present application by using a clinically used lung function detector in advance, to acquire a corresponding relationship between chest and abdomen volumes and lung volumes.

During specific calibration, the millimeter-wave radar and the clinical lung function detector are both used to detect the lung function of the subject under test, and two sets of detection data are obtained at the same time. The detection data given by the lung function detector is used as a gold standard for the lung volume. By fitting, machine learning, or the like, a conversion relationship between the chest and abdominal cavity volume measured with a non-contact lung function detection method based on the millimeter-wave radar and a gold standard value is established, that is, the calibration is completed.

As an example, linear or polynomial fitting may be performed between the chest and abdominal cavity volume and the lung volume to analyze the conversion relationship between the two. Alternatively, the conversion relationship between the chest and abdominal cavity volume and the lung volume is acquired through deep learning by collecting a large number of training samples.

It should be understood that, in some cases, the lung ventilation change information may be directly determined according to the lung volume change information, while in some other cases, the lung volume change information is required to be further processed before the lung ventilation change information of the subject under test can be analyzed, which is not limited in this embodiment.

In this embodiment, the lung function detection device models the chest and abdomen surface according to the chest and abdomen surface point cloud data, acquires changes in the chest and abdominal cavity volume of the subject under test over time, and then analyzes the lung ventilation change information according to the changes in the chest and abdominal cavity volume over time. In this way, the lung ventilation change information of the subject under test can be analyzed based on the chest and abdomen surface point cloud data acquired without contact and the pre-calibrated conversion relationship between the chest and abdominal cavity volume and the lung volume, which prevents detection errors that exist in contact detection on the subject under test, thereby improving accuracy of the detection result and detection safety.

In an embodiment, the analyzing the chest and abdomen surface point cloud data further includes acquiring chest and abdominal cavity morphology according to the chest and abdomen surface point cloud data; and the acquiring the lung ventilation information includes acquiring a conversion relationship among the chest and abdominal cavity volume, the chest and abdominal cavity morphology, and the lung volume; determining lung volume change information of the subj ect under test according to the chest and abdominal cavity volume curve, the chest and abdominal cavity morphology, and the conversion relationship; and determining the lung ventilation change information according to the lung volume change information.

In addition to an overall volume of a chest and abdominal cavity, morphological changes in the chest and abdominal cavity may also affect the lung ventilation. For example, in the case of a same chest and abdominal cavity volume, it is possible that in one case a chest cavity volume is larger and in the other case the abdominal cavity volume is larger. Therefore, the acquisition of the chest and abdominal cavity morphology through the chest and abdomen surface point cloud data can be used to further confirm the lung ventilation. Specifically, the acquiring the lung ventilation information includes acquiring a conversion relationship among the chest and abdominal cavity volume, the chest and abdominal cavity morphology, and the lung volume, lung volume change information of the subject under test may be determined according to the chest and abdominal cavity volume curve, the chest and abdominal cavity morphology, and the conversion relationship, and then the lung ventilation change information is determined according to the lung volume change information.

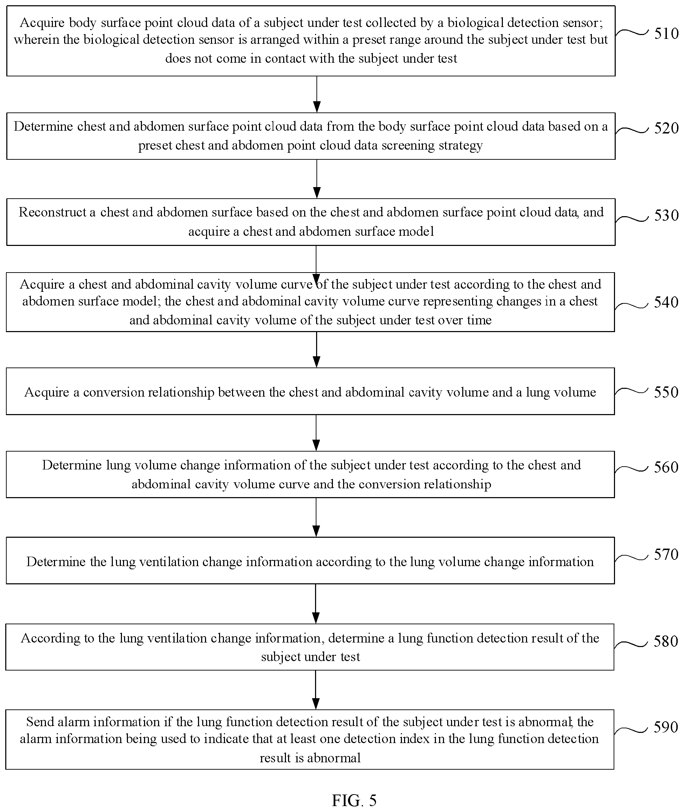

Based on the above embodiments, in an embodiment, as shown in FIG. 5, the present application further provides a lung function detection method. Description is also based on an example in which the method is applied to the lung function detection device in FIG. 1, including the following steps.

In step 510, body surface point cloud data of a subject under test collected by a biological detection sensor is acquired; wherein the biological detection sensor is arranged within a preset range around the subject under test but does not come in contact with the subject under test.

In step 520, chest and abdomen surface point cloud data is determined from the body surface point cloud data based on a preset chest and abdomen point cloud data screening strategy.

In step 530, a chest and abdomen surface is reconstructed based on the chest and abdomen surface point cloud data, and a chest and abdomen surface model is acquired.

In step 540, a chest and abdominal cavity volume curve of the subject under test is acquired according to the chest and abdomen surface model; and the chest and abdominal cavity volume curve represents changes in a chest and abdominal cavity volume of the subject under test over time.

In step 550, a conversion relationship between the chest and abdominal cavity volume and a lung volume is acquired.

In step 560, lung volume change information of the subject under test is determined according to the chest and abdominal cavity volume curve and the conversion relationship.

In step 570, the lung ventilation change information is determined according to the lung volume change information.

In step 580, according to the lung ventilation change information, a lung function detection result of the subject under test is determined.

In step 590, alarm information is sent if the lung function detection result of the subject under test is abnormal; and the alarm information is used to indicate that at least one detection index in the lung function detection result is abnormal.

Implementation principles and technical effects of the steps in the lung function detection method provided in this embodiment are similar to those in the previous method embodiments, which are not described in detail herein.

It should be understood that, although the steps in the flowcharts involved in the embodiments as described above are displayed in sequence as indicated by the arrows, the steps are not necessarily performed in sequence in the order indicated by the arrows. Unless otherwise clearly specified herein, the steps are performed without any strict sequence limitation, and may be performed in other orders. In addition, at least some steps in the flowcharts involved in the embodiments as described above may include a plurality of sub-steps or a plurality of stages, and such sub-steps or stages are not necessarily performed at a same moment, and may be performed at different moments. The sub-steps or stages are not necessarily performed in sequence, and the sub-steps or stages and at least some of other steps or sub-steps or stages of other steps may be performed in turn or alternately.

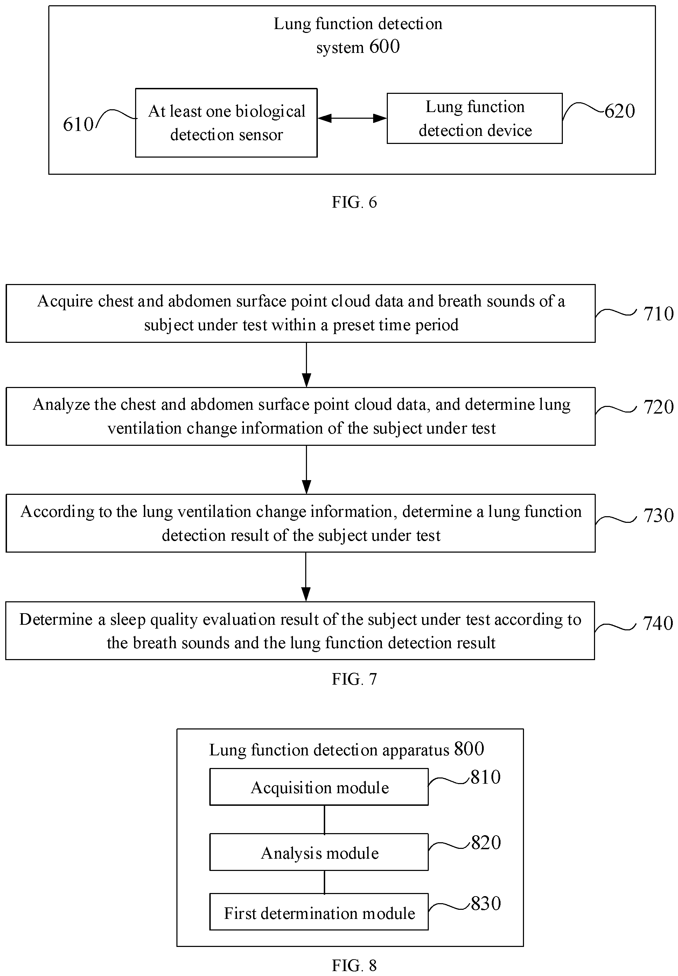

Based on a same inventive concept, in an embodiment, as shown in FIG. 6, the present application further provides a lung function detection system configured to implement the lung function detection method as described above. The lung function detection system 600 includes: at least one biological detection sensor 610 and a lung function detection device 620. The lung function detection device 620 is connected to the at least one biological detection sensor 610.

The at least one biological detection sensor 610 is configured to collect chest and abdomen surface point cloud data of a subject under test within a preset time period. The lung function detection device 620 is configured to analyze the chest and abdomen surface point cloud data, determine lung ventilation change information of the subject under test, and according to the lung ventilation change information, determine a lung function detection result of the subject under test.

In a possible implementation, the lung function detection device may include: functional modules such as a millimeter-wave radar module (in this case, the corresponding biological detection sensor is a millimeter-wave radar), a pickup module, a communication module, a power module, a control module, a computing module, and an interaction module. In actual application, the above functional modules may be deployed separately or integrated, so that the lung function detection device may include more or fewer functional modules, which is not limited in this embodiment.

Specifically, the millimeter-wave radar module is connected to at least one millimeter-wave radar, and uses a millimeter-wave radar system of multiple-input multiple-output (MIMO) transmission to acquire the chest and abdomen surface point cloud data of the subject under test. Each point in the chest and abdomen surface point cloud data represents a reflected signal from a corresponding position on a surface of the subject under test. Each point includes three-dimensional spatial coordinates, a velocity, and signal strength information. That is, in the present application, the millimeter-wave radar module is responsible for transmitting and receiving a millimeter-wave radar signal, generating point cloud data, filtering static point cloud, and other functions, and finally obtains the chest and abdomen surface point cloud data of the subject under test.

The pickup module is configured to collect breath sounds of the subject under test. Specifically, the breath sounds of the subject under test are collected through a microphone array, and the breath sounds are used as auxiliary information to detect and diagnose diseases.

The pickup module is not a mandatory module and may be deployed in the lung function detection device or may not be deployed.

In addition, the communication module is responsible for synchronization and communication between the primary radar and the secondary radar, between the radar and the computing module, between the pickup module and the computing module, and between the radar and the server, and transmits control commands and data. The control module controls a lung function detection process, including circuit timing, control logic, radar signal transmission and reception, parameter settings, and the like. The power module supplies power to the lung function detection device. The computing module is responsible for all radar data processing, including radar point cloud generation, point cloud extraction, body surface reconstruction, chest and abdominal cavity volume calculation and change tracking, conversion between the chest and abdominal cavity volume and the lung volume, and the like. The interaction module is configured to implement interaction between the user and the lung function detection device, such as voice control, infrared remote control, and mobile APP control. At the same time, the lung function detection device may output alarm information and detection data externally through voice output, a change in a color of an indicator light, an APP, or the like.

Further, when the lung function detection is performed on the subject under test in the above method embodiments, there are no requirements for the breathing pattern, that is, the subject under test may be detected when in a waking state or the subject under test may be detected when in a sleep state, which is not limited in the present application.

For example, the subject under test is a human body. Medical research shows that many major diseases of modern humans, such as hypertension, coronary heart diseases, arrhythmia, and diabetes, are typically related to sleep apnea-hypopnea syndrome (SAHS) that often occurs during sleep, making the SAHS a disease that seriously endangers people's lives and health. In addition, during sleep, many physiological functions inside the human body may undergo significant changes, such as a slowed heart rate, decreased blood pressure, slow metabolism, and a reduced number of breaths. Due to time continuity of a sleep process and changes in a body function regulation system, early signals of a variety of chronic diseases are often easy to catch during this period. There is a need to monitor and evaluate sleep quality of the human body to detect some potential diseases in advance.

However, sleep quality monitoring is mainly monitored by using polysomnography and a micro-motion sensitive mattress. The polysomnography measures relevant parameters by pasting electrodes on the human body, whose monitoring parameters mainly include 10 physiological signals such as electroencephalogram, electrocardiogram, electrooculogram, mandibular electromyography, oral and nasal respiratory airflows, chest and abdominal breathing movement, blood oxygen saturation, snoring, body positions, and tibialis anterior muscle. The micro-motion sensitive mattress converts micro-pressure into an electrical signal through a pressure sensor, which may monitor parameters such as breathing, pulse, and body movement, achieving no-load or low-load sleep monitoring. However, existing sleep disorder monitoring technologies are basically contact-based, which are required to be performed in hospitals and required to be performed by professional technicians. This brings great psychological pressure to patients and may also cause erroneous measurement results, and a monitoring device is also relatively expensive. In addition to the above contact-based methods, non-contact monitoring methods based on video and image processing may also be used. Such methods based on a video processing technology mainly uses an infrared thermal imager, but can only be used in medical research institutions and are difficult to popularize due to high prices.

Based on this, when deployed with the pickup module and provided with the microphone array, the lung function detection device in the present application may also be configured to monitor and evaluate sleep quality of the subject under test.

In an embodiment, breath sounds of the subject under test are collected if the subject under test is in a sleep state; and a sleep quality evaluation result of the subject under test is determined according to the breath sounds and the lung function detection result.

In a possible implementation, as shown in FIG. 7, description is based on an example in which the sleep quality monitoring method is applied to the lung function detection device in FIG. 1, including the following steps.

In step 710, chest and abdomen surface point cloud data and breath sounds of a subject under test within a preset time period are acquired.

The breath sounds include breathing or snoring of the subject under test.

In step 720, the chest and abdomen surface point cloud data is analyzed, and lung ventilation change information of the subject under test is determined.

In step 730, according to the lung ventilation change information, a lung function detection result of the subject under test is determined.

In step 740, a sleep quality evaluation result of the subject under test is determined according to the breath sounds and the lung function detection result.

Further, when apnea or an abnormal lung function detection result is detected, an alarm is made and alarm information is outputted.

In this embodiment, breathing and snoring of the subject under test are monitored by using a microphone array. At the same time, the chest and abdomen surface point cloud data of the subject under test is acquired by means of a biological detection sensor installed on a ceiling and corresponding to a sleeping bed of the subject under test, and sleep quality of the subject under test is comprehensively evaluated based on information such as breathing frequencies and lung ventilation. In this way, the sleep quality of the subject under test can be effectively evaluated without contact.

In other embodiments, other sensor data may be further introduced. For example, blood oxygen data, ECG data, and the like may be received from fingertip oxygen, watches, or the like, which are combined with a non-contact sensing module in this embodiment to evaluate and analyze sleep quality and cardiopulmonary functions.

Based on a same inventive concept, the embodiments of the present application further provide a lung function detection apparatus configured to implement the lung function detection method as described above. The implementation solution provided by the apparatus to solve the problem is similar to the implementation solution described in the above method. Therefore, specific limitations in the embodiments of one or more lung function detection apparatuses provided below may be obtained with reference to the limitations on the lung function detection method above. Details are not described herein.

In an embodiment, as shown in FIG. 8, a lung function detection apparatus is provided, including an acquisition module 810, an analysis module 820, and a first determination module 830.

The acquisition module 810 is configured to acquire chest and abdomen surface point cloud data of a subject under test within a preset time period by means of a biological detection sensor; wherein the biological detection sensor is arranged within a preset range around the subject under test but does not come in contact with the subject under test.

The analysis module 820 is configured to analyze the chest and abdomen surface point cloud data, and determine lung ventilation change information of the subject under test.

The first determination module 830 is configured to, according to the lung ventilation change information, determine a lung function detection result of the subject under test.

In an embodiment, the analysis module 820 includes:
a chest and abdominal cavity volume acquisition unit configured to acquire a chest and abdominal cavity volume curve of the subject under test according to the chest and abdomen surface point cloud data; the chest and abdominal cavity volume curve representing changes in a chest and abdominal cavity volume of the subject under test over time; and
a lung ventilation analysis unit configured to analyze the chest and abdominal cavity volume curve and acquire the lung ventilation change information.

In an embodiment, the chest and abdominal cavity volume acquisition unit includes:
a surface model reconstruction subunit configured to reconstruct a chest and abdomen surface based on the chest and abdomen surface point cloud data, and acquire a chest and abdomen surface model; and
a chest and abdominal cavity volume acquisition subunit configured to acquire the chest and abdominal cavity volume curve of the subject under test according to the chest and abdomen surface model.

In an embodiment, the lung ventilation analysis unit includes:
an acquisition subunit configured to acquire a conversion relationship between the chest and abdominal cavity volume and a lung volume;
a lung volume determination subunit configured to determine lung volume change information of the subject under test is determined according to the chest and abdominal cavity volume curve and the conversion relationship; and
a lung ventilation determination subunit configured to determine the lung ventilation change information according to the lung volume change information.

In an embodiment, the acquisition module 810 includes:
a point cloud acquisition unit configured to acquire body surface point cloud data of the subject under test collected by the biological detection sensor; and
a point cloud screening unit configured to determine the chest and abdomen surface point cloud data from the body surface point cloud data based on a preset chest and abdomen point cloud data screening strategy.

In an embodiment, the apparatus 800 further includes:
an alarm module configured to send alarm information if the lung function detection result of the subject under test is abnormal. The alarm information is used to indicate that at least one detection index in the lung function detection result is abnormal.

In an embodiment, the biological detection sensor is a radar, and the radar is arranged in front of the chest and abdomen of the subject under test.

In an embodiment, the radar includes a primary radar and a secondary radar, the primary radar is arranged in front of the chest and abdomen of the subject under test, and the secondary radar is arranged behind the back of the subject under test.

In an embodiment, the apparatus 800 further includes:
a collection module configured to collect breath sounds of the subject under test if the subject under test is in a sleep state; and
a second determination module configured to determine a sleep quality evaluation result of the subject under test according to the breath sounds and the lung function detection result.

Each module in the above lung function detection apparatus may be implemented entirely or partially by software, hardware, or a combination thereof. The foregoing modules may be built in or independent of a processor of a computer device in a form of hardware, or may be stored in a memory of the computer device in a form of software, so that the processor invokes and performs an operation corresponding to each of the foregoing modules.

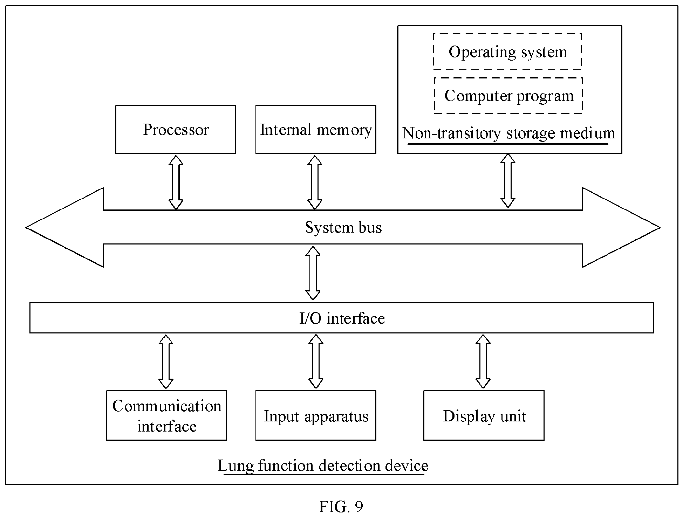

In an embodiment, a lung function detection device is provided. The lung function detection device may be a terminal device. An internal structure diagram thereof may be shown in FIG. 9. The lung function detection device includes a processor, a memory, a communication interface, a display screen, and an input apparatus connected through a system bus. The processor of the lung function detection device is configured to provide computing and control capabilities. The memory of the lung function detection device includes a non-transitory storage medium and an internal memory. The non-transitory storage medium stores an operating system and a computer program. The internal memory provides an environment for running of the operating system and the computer program in the non-transitory storage medium. The communication interface of the lung function detection device is configured for wired or wireless communication with an external terminal. The wireless communication may be realized by WIFI, a carrier network, near field communication (NFC), or other technologies. The computer program is executed by the processor to implement a lung function detection method. The display screen of the lung function detection device may be a liquid crystal display screen or an e-ink display screen. The input apparatus of the lung function detection device may be a touch layer covering the display screen, or may be a button, a trackball, or a touchpad disposed on a housing of the lung function detection device, or may be an external keyboard, touchpad, a mouse, or the like.

Those skilled in the art may understand that the structure shown in FIG. 9 is merely a block diagram of a partial structure related to a solution in the present application, and does not constitute a limitation to the lung function detection device to which the solution in the present application is applied. Specifically, the lung function detection device may include more or fewer components than those shown in the figure, or have some components combined, or have a different component deployment.

In an embodiment, a lung function detection device is provided, including a memory and a processor. The memory stores a computer program. The processor, when executing the computer program, implements the following steps:
acquiring chest and abdomen surface point cloud data of a subject under test within a preset time period by means of a biological detection sensor; wherein the biological detection sensor is arranged within a preset range around the subject under test but does not come in contact with the subject under test;
analyzing the chest and abdomen surface point cloud data, and determining lung ventilation change information of the subject under test; and
according to the lung ventilation change information, determining a lung function detection result of the subject under test.

Implementation principles and technical effects of the lung function detection device provided in the above embodiment are similar to those of the previous method embodiments, which are not described in detail herein.

In an embodiment, a computer-readable storage medium is provided, having a computer program stored therein. The computer program, when executed by a processor, implements the following steps:
acquiring chest and abdomen surface point cloud data of a subject under test within a preset time period by means of a biological detection sensor; wherein the biological detection sensor is arranged within a preset range around the subject under test but does not come in contact with the subject under test;
analyzing the chest and abdomen surface point cloud data, and determining lung ventilation change information of the subject under test; and
according to the lung ventilation change information, determining a lung function detection result of the subject under test.

Implementation principles and technical effects of the computer-readable storage medium provided in the above embodiment are similar to those of the previous method embodiments, which are not described in detail herein.

In an embodiment, a computer program product is provided, including a computer program. The computer program, when executed by a processor, implements the following steps:
acquiring chest and abdomen surface point cloud data of a subject under test within a preset time period by means of a biological detection sensor; wherein the biological detection sensor is arranged within a preset range around the subject under test but does not come in contact with the subject under test;
analyzing the chest and abdomen surface point cloud data, and determining lung ventilation change information of the subject under test; and
according to the lung ventilation change information, determining a lung function detection result of the subject under test.

Implementation principles and technical effects of the computer program product provided in the above embodiment are similar to those of the previous method embodiments, which are not described in detail herein.

It is to be noted that user information (including, but not limited, to user equipment information, user personal information, and the like) and data (including, but not limited to, data for analysis, stored data, displayed data, and the like) involved in the present application are all authorized by the user or information and data fully authorized by all parties.

According to the lung function detection method, system and apparatus, and the computer device and the storage medium in the present application, chest and abdomen surface point cloud data of a subject under test within a preset time period is acquired by means of a biological detection sensor; the chest and abdomen surface point cloud data is analyzed, and lung ventilation change information of the subject under test is determined; and according to the lung ventilation change information, a lung function detection result of the subject under test is determined. The biological detection sensor is arranged within a preset range around the subject under test but does not come in contact with the subject under test. In the present application, during the lung function detection on the subject under test, the lung ventilation change information is determined through the chest and abdomen surface point cloud data of the subject under test collected by the biological detection sensor. Compared with the related art in which the lung ventilation change information of the subject under test is detected by blowing air into a blowing nozzle, there are no requirements for the breathing pattern of the subject under test, which prevents detection errors caused by an unsatisfactory breathing rhythm of the subject under test, thereby improving accuracy of the detection result. In addition, the biological detection sensor in the present application is not in contact with the subject under test, and is not required to be in direct contact with the subject under test during the detection. For scenarios in hospitals where lung function detection is required to be performed sequentially on a plurality of subjects under test, non-contact detection may reduce a risk of cross infection and is safer.

Those of ordinary skill in the art may understand that all or some of procedures of the method in the foregoing embodiments may be implemented by a computer program instructing relevant hardware. The computer program may be stored in a non-transitory computer-readable storage medium. When the computer program is executed, the procedures of the foregoing method embodiments may be implemented. Any reference to a memory, storage, a database, or another medium used in the embodiments provided the present application may include at least one of a non-transitory memory and a transitory memory. The non-transitory memory may include a read-only memory (ROM), a magnetic tape, a floppy disk, a flash memory, an optical memory, and the like. The transitory memory may include a random access memory (RAM) or an external cache. By way of description and not limitation, the RAM may be in various forms, such as a static random access memory (SRAM), a dynamic random access memory (DRAM), or the like.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the specification.

The above-described embodiments are only several implementations of the present application, and the descriptions are relatively specific and detailed, but they should not be construed as limiting the scope of the invention. It should be understood by those of ordinary skill in the art that various modifications and improvements can be made without departing from the concept of the present application, and all fall within the protection scope of the present application. Therefore, the patent protection of the present application shall be defined by the appended claims.

## Claims

1. A lung function testing method, wherein the method comprises:
acquiring chest and abdomen surface point cloud data of a subject under test within a preset time period by means of a biological detection sensor; wherein the biological detection sensor is arranged within a preset range around the subject under test but does not come in contact with the subject under test;
analyzing the chest and abdomen surface point cloud data, and determining lung ventilation change information of the subject under test;
according to the lung ventilation change information, determining a lung function detection result of the subject under test.

2. The method according to claim 1, wherein the analyzing the chest and abdomen surface point cloud data, and determining lung ventilation change information of the subject under test comprises:
acquiring a chest and abdominal cavity volume curve of the subject under test according to the chest and abdomen surface point cloud data; the chest and abdominal cavity volume curve representing changes in a chest and abdominal cavity volume of the subject under test over time;
analyzing the chest and abdominal cavity volume curve, and acquiring the lung ventilation change information.

3. The method according to claim 2, wherein the acquiring a chest and abdominal cavity volume curve of the subject under test according to the chest and abdomen surface point cloud data comprises:
reconstructing a chest and abdomen surface based on the chest and abdomen surface point cloud data, and acquiring a chest and abdomen surface model;
acquiring the chest and abdominal cavity volume curve of the subject under test according to the chest and abdomen surface model.

4. The method according to claim 3, wherein the chest and abdomen surface point cloud data comprises coordinate information of three-dimensional spaces of respective point cloud points; and the reconstructing a chest and abdomen surface based on the chest and abdomen surface point cloud data, and acquiring a chest and abdomen surface model comprises: interpolating, based on the chest and abdomen surface point cloud data, three-dimensional coordinates of the point cloud points based on surface functions to generate point clouds and surface meshes, so as to acquire the chest and abdomen surface model.

5. The method according to claim 3, wherein the chest and abdomen surface point cloud data comprises coordinate information of three-dimensional spaces of respective point cloud points; and the reconstructing a chest and abdomen surface based on the chest and abdomen surface point cloud data, and acquiring a chest and abdomen surface model comprises: fitting, by using three-dimensional coordinate information of adjacent point cloud points in a polynomial or B-spline manner, a surface expression of a region where the point cloud points are located, to obtain a surface expression of the chest and abdomen surface, so as to acquire the chest and abdomen surface model.

6. The method according to claim 3, wherein the chest and abdomen surface point cloud data comprises coordinate information of three-dimensional spaces of respective point cloud points; and the reconstructing a chest and abdomen surface based on the chest and abdomen surface point cloud data, and acquiring a chest and abdomen surface model comprises:
pre-training a skinned multi-person linear model (SMPL) through data and deep learning;
inputting the coordinate information of the three-dimensional spaces of the respective point cloud points in the chest and abdomen surface point cloud data into the SMPL, and reconstructing body surface and posture information of a human body, so as to acquire the chest and abdomen surface model.

7. The method according to any one of claims 2 to 6, wherein the analyzing the chest and abdominal cavity volume curve, and acquiring the lung ventilation change information comprises:
acquiring a conversion relationship between the chest and abdominal cavity volume and a lung volume;
determining lung volume change information of the subject under test according to the chest and abdominal cavity volume curve and the conversion relationship;
determining the lung ventilation change information according to the lung volume change information.

8. The method according to any one of claims 2 to 6, wherein the analyzing the chest and abdomen surface point cloud data, and determining lung ventilation change information of the subject under test comprises:
acquiring chest and abdominal cavity morphology according to the chest and abdomen surface point cloud data;
acquiring a conversion relationship among the chest and abdominal cavity volume, the chest and abdominal cavity morphology, and the lung volume;
determining lung volume change information of the subject under test according to the chest and abdominal cavity volume curve, the chest and abdominal cavity morphology, and the conversion relationship;
determining the lung ventilation change information according to the lung volume change information.

9. The method according to any one of claims 1 to 8, wherein the acquiring chest and abdomen surface point cloud data of a subject under test within a preset time period by means of a biological detection sensor comprises:
acquiring body surface point cloud data of the subject under test collected by the biological detection sensor;
determining the chest and abdomen surface point cloud data from the body surface point cloud data based on a preset chest and abdomen point cloud data screening strategy.

10. The method according to claim 9, wherein the preset chest and abdomen point cloud data screening strategy is selected from at least one of distances between the point cloud points, moving velocities of the point cloud points, and three-dimensional spatial position coordinates of the point cloud points.

11. The method according to any one of claims 1 to 10, wherein the method further comprises:
sending alarm information if the lung function detection result of the subject under test is abnormal; the alarm information being used to indicate that at least one detection index in the lung function detection result is abnormal.

12. The method according to any one of claims 1 to 11, wherein the biological detection sensor is a radar, the radar being arranged in front of the chest and abdomen of the subject under test.

13. The method according to claim 12, wherein the radar comprises a primary radar and a secondary radar, the primary radar being arranged in front of the chest and abdomen of the subject under test, and the secondary radar being arranged behind the back of the subject under test.

14. The method according to any one of claims 1 to 13, wherein the method further comprises:
collecting breath sounds of the subject under test if the subject under test is in a sleep state;
determining a sleep quality evaluation result of the subject under test according to the breath sounds and the lung function detection result.

15. The method according to any one of claims 1 to 14, wherein the chest and abdomen surface point cloud data comprises position information of respective points in chest and abdomen of the subject under test to represent rises and falls of the chest and abdomen of the subject under test caused by breathing.

16. A lung function detection system, wherein the system comprises at least one biological detection sensor and a lung function detection device, the lung function detection device being connected to the at least one biological detection sensor;
the biological detection sensor being configured to collect chest and abdomen surface point cloud data of a subject under test within a preset time period;
the lung function detection device being configured to analyze the chest and abdomen surface point cloud data collected by the biological detection sensor, determine lung ventilation change information of the subject under test, and according to the lung ventilation change information, determine a lung function detection result of the subject under test.

17. The system according to claim 16, wherein the lung function detection device comprises a pickup module;
the pickup module being configured to collect breath sounds of the subject under test.

18. The system according to claim 17, wherein the lung function detection device is further configured to determine a sleep quality evaluation result of the subject under test according to the breath sounds and the lung function detection result.

19. A lung function detection apparatus, wherein the apparatus comprises:
an acquisition module configured to acquire chest and abdomen surface point cloud data of a subject under test within a preset time period by means of a biological detection sensor; wherein the biological detection sensor is arranged within a preset range around the subject under test but does not come in contact with the subject under test;
an analysis module configured to analyze the chest and abdomen surface point cloud data, and determine lung ventilation change information of the subject under test;
a first determination module configured to, according to the lung ventilation change information, determine a lung function detection result of the subject under test.

20. A lung function detection device, comprising a memory and a processor, the memory storing a computer program, wherein the processor, when executing the computer program, implements steps of the method according to any one of claims 1 to 15.

21. A computer-readable storage medium, having a computer program stored therein, wherein the computer program, when executed by a processor, implements steps of the method according to any one of claims 1 to 15.
